# EUROPEAN PATENT APPLICATION

(11) **EP 4 378 446 A1**
(43) Date of publication of application: **05.06.2024**
(21) Application number: 23212287.9
(22) Date of filing: 27.11.2023
(51) Int. Cl.: A61K 8/02, A61Q 1/06, A61K 8/34, A61K 8/37, A61K 8/42, A61K 8/44, A61K 8/81

(54) **COSMETIC ARTICLE IN STICK FORM AND A METHOD FOR MAKING IT**

(30) Priority: 29.11.2022 IT 202200024546
(71) Applicant: Chromavis S.p.A., 26010 Offanengo (CR) (IT)
(72) Inventor: Guizzetti, Giorgio, VIMERCATE (MB) (IT); Gaboardi, Mauro, Pizzighettone (CR) (IT); Raffaldi, Samuele, Fombio (LO) (IT); Ruffoni, Gianluca Nazzareno, Ripalta Cremasca (CR) (IT)
(74) Representative: Vanosi, Adelio Valeriano

(57) **Abstract**

The present invention describes a cosmetic article, in particular for make-up, in stick form, comprising a transparent external product and a liquid internal product within which solid decorative elements are dispersed. The cosmetic article features an attractive, original appearance, which can be appreciated from both an aesthetic and a functional point of view upon application thereof to the skin.

## Description

### FIELD OF THE INVENTION

The present invention relates to a cosmetic article, in particular for make-up, and to a method for making it.

Specifically, the invention relates to an article in stick form, for example with a circular section and a diameter of 10 mm upwards, or with any section (square, rectangular, star-shaped, possibly with rounded corners, etc.) such as a product for lips, eyes, face, and body, for example and in more detail: a lipstick, a lip balm, a foundation, a concealer, a primer, an eye shadow, a blusher, a highlighter, a bronzer, or a deodorant.

### BACKGROUND ART

Many cosmetic articles, in particular articles for make-up, are marketed in stick form, i.e. in the form of a small cylinder or small tube with a twist-up and down mechanism, and are therefore solid (think of lipsticks, lip balms, foundations, and deodorants, among others). This format has the advantage of making the cosmetic easier to use, as you can apply it more accurately in the desired area, without touching the cosmetic product with your hands.

In order to attract consumers, in addition to improving the functionality of these articles, there is increasing attention to the aesthetics thereof. Over time, therefore, cosmetic articles have been designed which are increasingly attractive to the consumer and/or which create particular aesthetic effects both before and following application.

Influenced by constantly changing trends, consumers are increasingly exacting and, indeed, look for products which are innovative, especially for make-up, provide original or specific visual effects, and are at the same time eye-catching, in terms of the aesthetics of the said article, in order to encourage people to choose and purchase them.

Stick cosmetics that meet these needs are, for example, lipsticks with a shimmering effect (also referred to as "glitter") obtained by incorporating fragments of various iridescent or pearlescent materials into the bulk product. There are also varyingly transparent cosmetic sticks available on the market that include decorative elements in different shapes, for example in powder form or in other forms and shapes such as a flower, heart, star etc., which are visible externally.

There are also commonly known products, such as lip gloss, i.e. a transparent gel featuring varying degrees of colour, within which the said glitter may be dispersed. Depending on the consistency of the gel, lip gloss can be produced in various forms. In particular, more liquid formulations can be packaged in a bottle, for example, equipped with a soft, fluffy applicator or with a roll-on applicator, or in a rigid case (made of glass or plastic) with a wand applicator. The more solid formulations may be presented in the form of sticks with various diameters or contained in a jar. To apply the latter form, it is necessary to use a suitable applicator (brush, sponge, etc.), , or dab your fingers on the product and then apply it in the desired area.

Although the latter is clearly more attractive from an aesthetic and commercial point of view, it must nevertheless be considered that more liquid lip glosses are also those that tend to stay on less, because - unlike lipsticks - they generally do not have a waxy structure.

### SUMMARY OF THE INVENTION

The object according to the present invention is to produce a cosmetic article, in particular for make-up or skin care, which is improved with respect to the known art.

This and other objects are achieved by producing a cosmetic article, in particular for make-up according to the technical teachings of the claims annexed hereto.

One advantage of the cosmetic article, in particular for make-up, according to the invention is therefore to offer eye-catching aesthetic properties through the use of decorative elements, which are rendered dynamic by the suspension thereof in a fluid product which, nevertheless, maintains the advantages of solid products in stick form.

A further advantage is the possibility of incorporating, using the stick as a carrier, water-soluble active ingredients or colorants in an aqueous matrix or fat-soluble active ingredients or colorants in an oily matrix.

A still further advantage is that a stick product can be obtained that combines a liquid part therewithin in order to benefit from the characteristics of both a solid waxy product and a liquid one.

### BRIEF DESCRIPTION OF THE FIGURES

Further characteristics and advantages of the invention will become apparent in the description of a preferred but not exclusive embodiment of the invention, illustrated - by way of a non-limiting example - in the drawings annexed hereto, in which:
Figure 1 is a schematic side view and sectional view of a mould which can be used to form the stick;
Figures 2 to 7 show schematically, and again from a sectional view, various steps in the implementation of the invention;
Figure 8 shows a cross sectional view, taken along the line VIII-VIII in Figure 11, of the stick according to the present invention;
Figure 9 is a top-down view of the stick in Figure 11;
Figure 10 is a longitudinal sectional view of the stick taken along line VII-VII in Figure 11;
Figure 11 is a front view of the stick according to the present invention;
Figure 12 shows six possible variants, schematically and from a cross-sectional view, of a cavity in the stick according to the present invention; and
Figures 13, 14, and 15 show three different cross-sectional views of possible variants of the stick according to the invention.

### DETAILED DESCRIPTION OF THE INVENTION

The invention relates to a cosmetic article 20, preferably for make-up, in stick form.

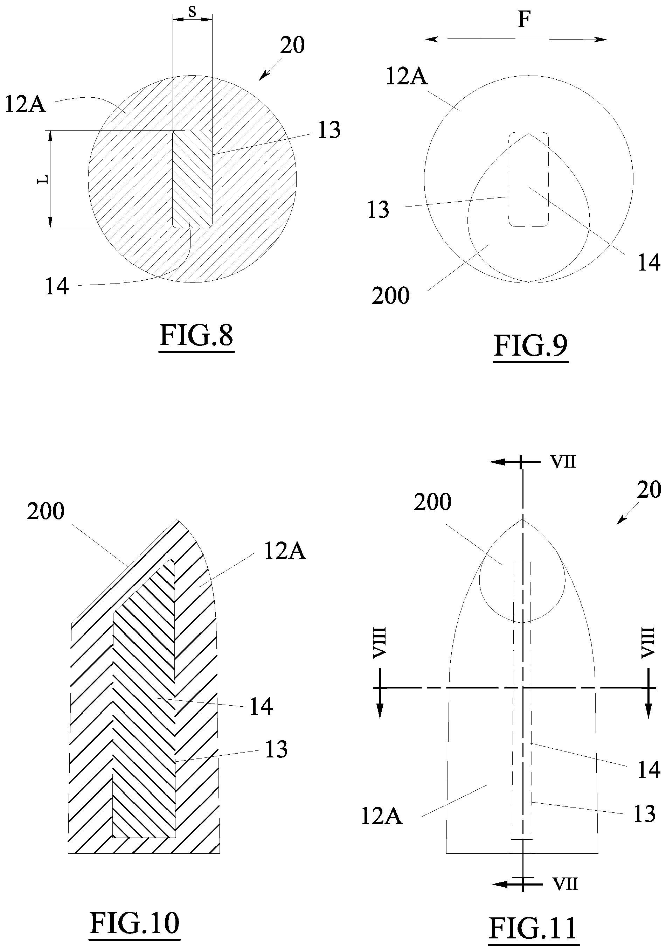

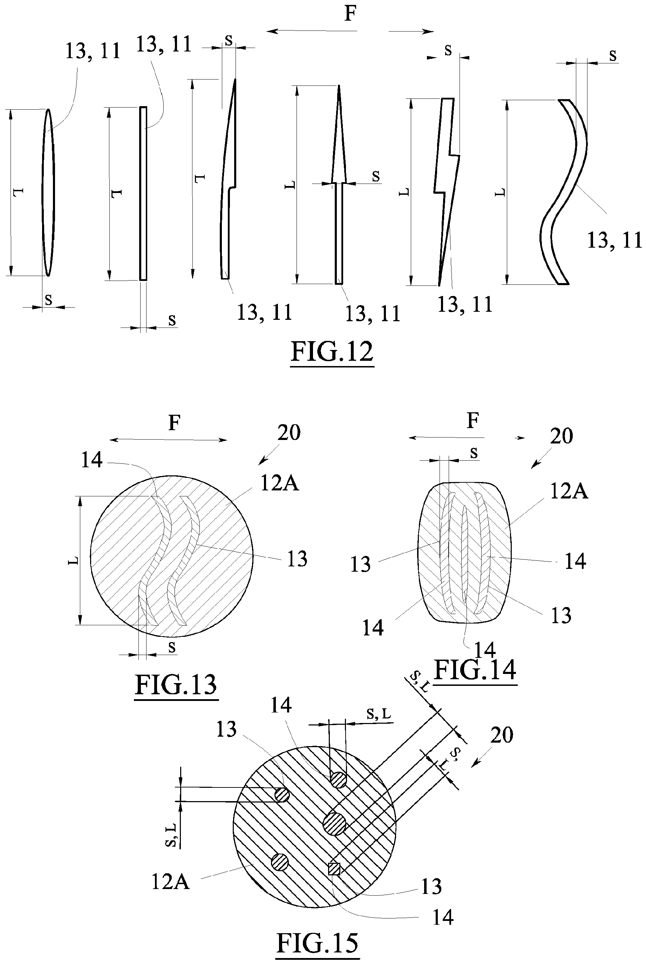

Specifically, it relates to an article in the form of a stick, for example with a circular section - Figs. 9 or 13 - and a diameter of between 8 mm and 50 mm, or with any section (square, rectangular, star-shaped, possibly with rounded corners - Fig. 14 - etc.) such as a product for lips, eyes, face, and body, for example and in more detail: a lipstick, a lip balm, a foundation, a concealer, a primer, an eye shadow, a blusher, a highlighter, a bronzer, or a deodorant.

The stick 20 can have an essentially cylindrical or lowly conical shape. Advantageously, the stick can have a section which is tapered towards one of the end surfaces thereof, which is also useful to guarantee a correct demoulding angle.

As is visible in Figure 11, the stick can feature, at one of the free ends thereof, a tip with an essentially flat part 200 which has the purpose of determining, for the end user, a surface for the first application of the cosmetic article.

Specifically, a user, upon first use, will be able to place the essentially flat part 200 on their lips (in the case of a lipstick, lip gloss, lip balm) or on their skin (in the case of another cosmetic product).

The application of the product, conventionally, will proceed along a preferred application direction which is essentially shown by the arrow F.

The preferred application direction can be dictated by the conformation and position of the essentially flat part 200.

Application is possible in both directions, i.e., to the right or to the left, as in Figure 11, without distinction.

The cosmetic article 20 includes:
- a first cosmetic product 12A, in the form of a stick with an essentially solid or pasty consistency at room temperature, which delimits at least one internal cavity 13; and
- a second cosmetic product 14 which is liquid at room temperature and is housed in at least one internal cavity 13.

In the present document, the term 'liquid at room temperature' means that the second cosmetic product has a dynamic viscosity (at room temperature) of between 1 cPs and 100,000 cPs, more preferably between 1cPs and 10,000 cPs, even more preferably between 1 cPs and 1,000 cPs.

It should be noted that the closer the viscosity gets to 1 cPs (i.e. approximately the viscosity of water at 25°C), the more visible a possible "floating" effect of the suspended particles will be.

The aforesaid dynamic viscosity measurement is calculated at 25°C with a Brookfield Viscometer with a Helipath stand. For example with a Brookfield AMETEK DV2T viscometer.

The internal cavity 13 has a cross section with a conformation such as to close up mechanically during use of the cosmetic article 20 (for example when the article is used by moving it along the preferred application direction), thereby minimising or preventing the second cosmetic product 14 (liquid) leak from the said cavity after use of the stick.

To make this mechanically achieved (by sliding) closure possible, the cross section of the internal cavity 13, i.e., that perpendicular to an axis A of the cosmetic article 20, can have a very low thickness S, with a maximum width of between 1.0 and 3.5 mm, preferably between 1.8 and 2.8 mm, even more preferably 2.2 mm.

In this way, during the mechanical (sliding) action, deriving from use of the cosmetic article 20 in the main application direction F, part of the first (pasty/waxy) cosmetic product 12A melts/softens upon contact with the temperature of the body/lips (approximately 36°C), and is distributed over the entire application surface, penetrating (and thereby closing up) the cavity 13 which contains the second (liquid) cosmetic product 14. In this case, leakage of the second product 14 is prevented (or minimised).

This result is linked to the low thickness S (or width) of the cavity 13, which allows easy closing following penetration of the first product, once softened, into the end part of the cavity 13, facing the surface 20.

Conversely, the length L of the cavity (measured in an essentially orthogonal direction to the main application direction) has no influence on the closing of the cavity 13, therefore, only the maximum thickness S (measured parallel to the preferred application direction) is taken into account.

The preferred application direction F can be determined not only by the conformation and positioning of the essentially flat part 200 (which may also be absent), but also by the conformation of the cavity or cavities 13.

Indeed, the cavity 13 can feature (in all possible conformations, see also Figure 12) a maximum longitudinal extension (length L) and a maximum transverse extension (thickness or width S), the maximum longitudinal extension L being greater than (or equal to) the maximum transverse extension S.

Preferably the ratio between the maximum longitudinal extension (length L) and a maximum transverse extension (thickness or width S) is comprised between 5,1 and 2, more preferably between 4 and 2,5, even more preferably between 3,9 and 3.

In fact, it is preferred to have an internal cavity with an elongated conformation.

It has been verified that the distance between the outer lateral perimeter of the stick and the outer perimeter of the cavity is an important parameter; it may be at least 1,5mm, preferably at least 2mm in order to avoid the leaking of the liquid contained in the cavity on the lateral perimeter of the stick, during the use of the stick, and the breaking of the stick during its use.

The stick may have an outer diameter (if it has a circular cross shape or maximum diameter in case that the cross section of the stick is triangular, square, rectangular etc.) between 9 and 22mm, preferably between 11 and 18mm, more preferably of 12,7mm.

The preferred application direction will therefore be essentially orthogonal to the axis along which the maximum longitudinal extension L runs.

In this document, the term 'essentially orthogonal' means that the angle formed with respect to the axis along which the maximum longitudinal extension L runs is between 45° and 135°.

Obviously, one or more cavities 13 can also be created, as shown in Figures 13-15, having different shapes.

It must be said that each cavity 13 can be filled with a second cosmetic product 14 which is liquid (at room temperature) having different characteristics. For example, a cavity 13 can be filled with a second cosmetic product 14 with one colour (or which guarantees a certain effect), and another cavity can be filled with a second cosmetic product 14 with a different colour or effect.

An extreme case of the invention is shown in Figure 15. In this embodiment, the length L and the thickness S of the cavity 13 (note that there are multiple cavities) are identical or in any case comparable, and are included in the dimensions stated above in relation to the thickness S. In this case, there is no preferred application direction given that the cavities 13 close up automatically (during use) whatever the said main application direction is precisely because of their low thickness S or width L.

From the sectional view in Figure 10, it can be seen that the cavity 13 can be totally surrounded by the first cosmetic product 12A, or by one or more products with an essentially solid or pasty consistency at room temperature.

Advantageously, the second cosmetic product 14 can contain solid decorative elements (including floating ones), or fat-soluble dyes, or a combination thereof.

As is visible in Figure 10, the cavity 13 can extend to at least half the height A1 of the said stick.

Therefore, a height A2 of the cavity is less than the total height A1 of the stick.

Furthermore, the surface area of the cavity 13, in a cross-sectional view (for example as seen in Figure 8), is less than the surface area, in a cross-sectional view, of the said first product.

The first cosmetic product 12A, at room temperature, is preferably transparent or translucent so as to render the contents of the said internal cavity 13 visible from the outside to a user.

The first cosmetic product 12A can be a normal and conventional product, i.e., solid or pasty at room temperature, which has already been used for the production of cosmetic sticks, for example for lips.

The mechanical closure of the cavity 13 becomes particularly effective when the first cosmetic product 12A has a dropping point comprised between 100 and 120°C, preferably between 111 and 115°C.

The dropping point is calculated using a Mettler-Toledo DP-70 apparatus, with a temperature increase rate of 2°C/min, and a waiting time of 120 seconds. When the first drop falls, the measuring is stopped.

Also, the closure of the cavity is particularly effective when the breaking load of the first cosmetic product 12A is comprised between 800 and 1000 gf (gram force).

The dynamometer used for the test is a MADA model MX-500 N, with force gauge model DS2-50N.

A chisel tip of 8mm diameter is used on the dynamometer (A4, 1.9G) and the sample of the final dimension of the stick is maintained at 25°C.

The tip is pushed against the sample until the sample breaks. The speed of the tip is 0,22cm/s.

The first cosmetic product 12, 12A and the second cosmetic product 14, 14A are preferably make-up products, but can also be functional products, i.e., containing active ingredients.

In the following description, when referring to the first cosmetic product 12, 12A, the reference 12 will be used when the said product is in liquid or in any case fluid form (so as to be pourable), and therefore well above room temperature (for example, at 65 °C).

Reference number 12A will be used, however, when the first product is at room temperature, therefore in solid or paste form.

However, when referring to the second cosmetic product, reference number 14 will be used to denote the said product in liquid form, or to denote the said product in solid/gel form, for example after the said product has formed a structure due to a rise in temperature.

The term "room temperature" here means a temperature of 20-25 °C, preferably of 25 °C.

Room temperature is also considered the optimal temperature for use. Preferred storage conditions are also determined as between 10 °C and 45 °C.

It has therefore surprisingly been found that it is possible to overcome the aforesaid technical difficulty by creating cosmetic articles in stick form in which a first cosmetic product 12A, preferably for make-up or skin care, in solid or paste form at room temperature (bulk) forms, at least in part, the outermost portion of the article and delimits, at least partially, a cavity 13 containing a second product 14 in a liquid state, in which solid decorative elements, or a fat-soluble dye, or a combination thereof can be dispersed and can move freely.

The second product 14 is mechanically sealed within the cavity 13 each time the product is used, due to the very shape of the said cavity 13, in particular the low thickness thereof.

The possibility of mechanically sealing the cavity 13 allows the aesthetic effect of movement of the decorative elements and/or droplets of fat-soluble dye dispersed in a liquid matrix to be exploited while also preventing the latter from coming out when, during the application of the stick, the end of the cavity 13 is opened to allow the elements contained therein to come out.

Any droplets of product that could escape during opening during use of the stick are in any case retained by the surface tension of the second cosmetic product 14.

By increasing the use temperature, the viscosity of the liquid part decreases and at the same time the "melted" part of the pasty/waxy product increases, which will then seal the opening even more easily.

Therefore, an article has been advantageously obtained which combines the properties and practicality of use of a conventional stick, such as a transparent/semitransparent lipstick, with a dynamic aesthetic effect, typical of a liquid formulation (e.g. a conventional lip gloss in which glitter is dispersed, floating freely therein) and/or a functional effect linked to the insertion into the containment chamber (or cavity 13) of fat-soluble dyes which form coloured droplets upon contact with the aqueous matrix.

In this way, in the article according to the invention, the second product 14 contained in the cavity 13 of the body inside the first product 12, 12A, is in a liquid state at room temperature, and the dispersed decorative elements/ dyes (whether fat-soluble or not) can be suspended and move therein.

During use of the article, i.e., during application to the skin or lips, as the first product is used up and becomes thinner, allowing the second product to emerge, a combined (possibly make-up) effect of the two products is achieved.

In the meantime, the application movement spreads the first product over the application surface, mechanically sealing the cavity or cavities 13.

Preferably, the second cosmetic product 14, 14A comprises 50-95% by weight of water, based on the weight of the second product.

Optionally, the second product 14 further comprises a water-miscible solvent, more preferably an alcoholic solvent, such as ethanol.

As mentioned, the second product 14 also comprises solid decorative elements, or fat-soluble dye, or a combination thereof.

Preferably, the second product 14 comprises up to 35% by weight (based on the weight of the second product) of solid decorative elements, or fat-soluble dye, or a combination thereof, and more preferably 1-30% by weight.

The term "solid decorative element" is understood as comprising decorative elements such as pigments, as they are or on solid media, in small spheres, or glitter.

The term "spheres" (or "small spheres") refers not only to perfectly spherical solid forms but also solid forms that are essentially spherical or are akin to spheres, such as spheroids.

Preferably, the solid decorative elements dispersed in the second product can have essentially the same diameter or the same shape or different diameters or shapes. The choice depends on the aesthetic effect you want to achieve for the final article.

The said solid decorative elements can also be pearlescent pigments made on substrates, such as preferably: natural or synthetic mica (fluorphlogopite), calcium sodium borosilicate, calcium aluminium borosilicate, calcium titanium borosilicate, silica.

The said solid decorative elements can also be, as mentioned, glitter, for example synthetic glitter made of: polyethylene terephthalate, isobutylphenoxy epoxy resin/acrylate copolymer, polyethylene terephthalate/ polybutylene terephthalate, isobutylphenoxy epoxy resin, polyethylene terephthalate/polyurethane-11, polyethylene terephthalate, or natural glitter (bioglitter), for example bioglitter made of: rayon, cellulose acetate.

The said solid decorative elements can be pigments or pigment substrates such as: iron oxides (INCI:CI 77492, CI 77499, CI 77491), titanium oxide (CI 77891), manganese violet (CI 77742), ultramarine (CI 77007), chromium oxide green (CI 77288), chromium hydroxide green (INCI: CI 77289), sodium salt red 6 (CI 15850), yellow lake 5 (CI 19140), red lake 22 (CI 45380), red lake 21 (CI 45380), red 7 (CI 15850), red lake 7 (CI 15850), red lake 40 (CI 16035), carmine (CI 75470), red lake 28 (CI 45410), red lake 27 (CI 45410), yellow lake 6 (CI 15985), ferrous ammonium ferrocyanide (CI 77510), red lake 30 (CI 73360), blue lake 1 (CI 42090), aluminium powder (CI 77000) in pure form or dispersed in carriers of mineral origin such as talc or mica.

The term "fat-soluble dye" refers to one or more lipophilic dyes which, as such, are insoluble in water and therefore form coloured droplets therewithin.

Suitable fat-soluble dyes can be either of natural origin, such as castor oil, or of synthetic origin, such as octyldodecanol and octyldodecyl stearoyl stearate.

Optionally, water-soluble dyes can also be added to the water in the second product 14, which can therefore create a pleasant colour contrast with the solid decorative elements, or with the fat-soluble dye, or with a combination thereof.

Suitable water-soluble dyes include, for example, CI 19140 [YELLOW 5]100%, CI 15985 [YELLOW 6]100%, CI 42090 [BLUE 1]100%, CI 17200 [RED 33]100%, CI 45410 [RED 27] 100%, CI 45410 [RED 28] 100%, CI 45380 [RED 22]100%, CI 45380 [RED 21]100%).

In preferred embodiments, the second product 14 further comprises water-soluble active ingredients, such as vitamins (in particular vitamin C), botanical extracts, antimicrobial agents, anti-irritation agents, humectant agents, antioxidant agents, hydrating agents, absorbent agents of UV rays, and mixtures thereof.

Optionally, the second cosmetic product 14 is devoid of thermosensitive viscosifying agents.

According to particularly preferred aspects, the first product 12 is transparent or translucent, and may be coloured or non-coloured.

Preferably, the said first product is a solid gel comprising a lipid matrix and a gelling agent, capable of forming a network of microscopic-sized fibres in the lipid matrix, producing a solid and completely transparent gel during cooling.

Preferably, the said lipid matrix may comprise, comprises, or essentially consists of, one or more of the following: tridecyl trimellitate, hydrogenated polyisobutene and/or octyldodecanol.

Obviously, there may also be other types of esters, waxes, or film-forming agents typical of an anhydrous formulation present.

Preferably, the said gelling agent comprises, or essentially consists of, a mixture of the following: dibutyl ethylhexanoyl glutamide, dibutyl lauroyl glutamide, dispersed in the lipid matrix, preferably in a lipid matrix comprising, or consisting of, a polar ester, such as for example an octyldecanol ester.

Preferably, the said first product further comprises, either dissolved or suspended in said lipid gel, one or more of the following: lipophilic active ingredients, flavours, aromas, suspended elements, silica and derivatives, and pigments. The latter can be as described above with reference to the second product.

Optionally, the said first product 12 can further comprise at least one water-miscible solvent, more preferably an alcoholic solvent, such as ethanol or glycols.

Particularly preferred cosmetic articles in stick form according to the invention include: sticks for lips, such as lipsticks, lip balms, or lip glosses, eye and face sticks, such as eye shadows, foundations, concealers, primers, bronzers, blushers, face highlighters, and sticks for the body, such as deodorants.

The physical properties of the second product 14 are also particularly advantageous for the preparation of the article 20 according to the invention as the second product can be advantageously poured into the internal cavity 13 in the first solidified product 12A, the said second product being liquid at room temperature, as described in detail later on.

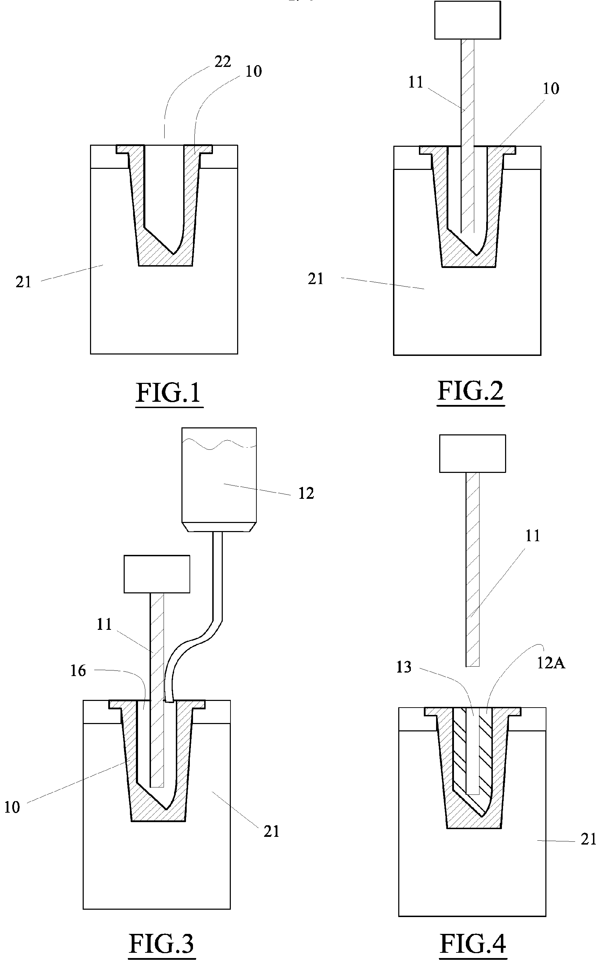

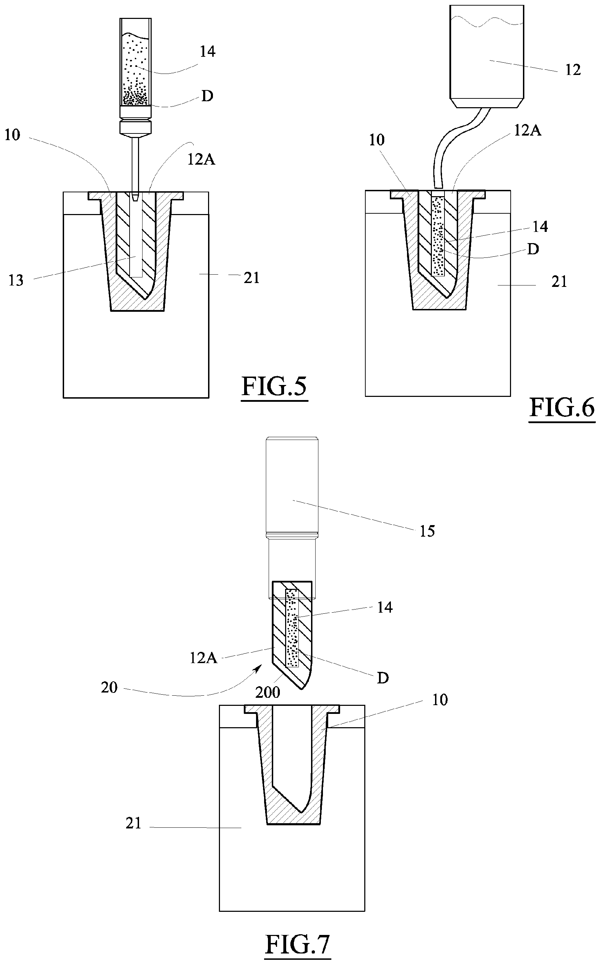

From a different perspective, the present invention also relates to a process for preparing the cosmetic article, preferably for make-up, as described above. This process comprises the following steps:
i. providing a mould 10 comprising a cavity 22 (Fig. 1),
ii. inserting a core 11 into the mould 10 cavity 22, so as to determine a first casting cavity 16 between the walls of the mould 10 delimiting the cavity 22 and the external perimeter of the core 11 (Fig. 2) and advantageously, the core 11 is not in contact with any part of the mould 10,
iii. pouring a first cosmetic product 12 into the first casting cavity 16 and waiting until the said first product has solidified (Fig. 3),
iv. once the first cosmetic product 12 has solidified, extracting (Fig. 4) the core 11, so that the portion of the first solidified product 12A which housed the core 11 determines an internal cavity 13 having a cross section sized so as to close up mechanically during use of the cosmetic article 20, thereby minimising or preventing the second cosmetic product 14 leaking from the said cavity after use of the cosmetic product,
v. pouring (Fig. 5) a second cosmetic product 14, in essentially liquid form into the internal cavity 13,
vi. isolating the second liquid product 14 inside the internal cavity 13, and
vii. removing the cosmetic article 20 from the mould.

Advantageously, the second liquid product 14 inside the cavity 13 is isolated by pouring on top thereof a product which takes on an essentially solid or pasty consistency at room temperature, preferably the first product 12 after heating until it takes on a liquid consistency.

The internal cavity 13 can have a cross section shaped in one of the following geometric forms: circle, ellipse, triangle, square, rectangle, heart-shaped, star-shaped, also with rounded corners, and other as shown in Figure 12.

Advantageously, the core 11 is sprinkled with a mould-release agent before being inserted into the mould 10.

The mould 10 can advantageously be made (at least partially) of silicone or another material suitable to form a stick (for example another elastic material, but also metal and/or ceramic).

The said mould can be housed or positioned in a vacuum chamber 21, which can apply a vacuum to the exterior of the mould in order to allow easy demoulding of a stick 20 made using the mould 10.

Before insertion into the mould 10, the core 11 can be covered with a release agent, or the said core can be made of a self-lubricating material that allows easy extraction thereof to form the internal cavity 13.

It goes without saying that the core 11 is configured to create the internal cavity 13 according to the specifications described above.

The first cosmetic product 12 can be heated to above room temperature before pouring, so as to take on an essentially liquid consistency or in any case a consistency that renders the said product easily pourable. For example, the first cosmetic product 12 is heated to approximately 65°C.

The first cosmetic product 12 inside the mould 10 can be cooled (for example by cold air) to below room temperature, to accelerate the solidification thereof.

The second cosmetic product 14 can be isolated within the internal cavity 13 by having one of the surfaces thereof facing - and therefore exposed to - a heat source (if the viscosifying agent is present) so as to render a layer of the second cosmetic product solid, or in any case to solidify the exposed surface.

Preferably, it is possible to pour (Fig. 6) the first liquid cosmetic product 12 or another product that is solid at room temperature onto at least an exposed surface of the second cosmetic product 14.

The removal of the stick 20 from the mould can be carried out according to conventional methods, for example by placing under vacuum inside the vacuum chamber 21.

Before or during demoulding, it is possible to couple the stick 20 to a device 15 (for example, for lipsticks or deodorants).

Obviously, the internal cavity 13 can assume a configuration corresponding to that of the core 11.

The said configuration can have a cross section shaped in one of the following geometric forms: circle, ellipse, triangle, square, rectangle, heart shape, star shape etc.

Some possible cross-sections of the cavity 13 (and therefore also of the core 11) are shown in Figure 12, which shows six alternatives. In this figure, the cross-section lines have been omitted for the sake of clarity.

The internal cavity 13 can extend to almost the final height A1 of the stick 20.

The wall of the stick surrounding the cavity or cavities 13 (formed by the first solidified cosmetic product 12A) will advantageously be thick enough to be able to effectively contain the second cosmetic product 14, liquid at room temperature, without breaking or ruining during normal use of the said stick 20.

Figures 1-7 show the steps for the preparation of a lipstick in stick form (therefore, in this case, the cosmetic article is a make-up article), according to preferred aspects of the present invention.

It should also be understood that all aspects identified as preferred and advantageous for the cosmetic article should be deemed equally preferable and advantageous also for the uses and the preparation process thereof.

It should likewise be understood that all combinations of the preferred aspects of the cosmetic article according to the invention, as well as the uses and the preparation process thereof, as set out above, should be considered described herein.

The following section provides embodiment examples of the present invention for illustration purposes.

### EXAMPLES

### Example 1.

A lipstick has been prepared according to the present invention.

A first product 12 comprising the composition below was poured into the first casting cavity 16 of a mould 10, between the internal walls of the mould 10 and the external perimeter of a core 11 (Fig. 3):

| ingredient | % by weight |
|---|---|
| OCTYLDODECANOL | 23 |
| DIBUTYL LAUROYL GLUTAMIDE | 3.50 |
| DIBUTYL ETHYLHEXANOYL GLUTAMIDE | 2.3 |
| TRIDECYL TRIMELLITATE | 39.2 |
| HYDROGENATED POLYISOBUTENE | 32 |
| Total | 100 |

The first product 12 was heated to a sufficient temperature to assume a liquid and therefore pourable consistency. Specifically, the first product was heated to 65 °C.

The core 11 has a lamellar shape, with a rectangular section and a thickness of 2.3 mm. The core is configured so as to obtain a cavity with the specifications described above, therefore with a thickness of 2.2 mm.

Once the first product 12A had solidified, the core 11 was extracted (Fig. 4) and a second product 14 comprising the composition stated below was poured (Fig. 5) into the internal cavity 13 thus formed (with a configuration corresponding to that of the core):

| ingredient | % by weight |
|---|---|
| Water | 93 |
| 1,2-hexanediol | 1.5 |
| Hydroxyacetophenone | 0.5 |
| Pigmented glitter/Dispersed beads | 5 |
| Total | 100 |

The second product 14 was then isolated (Fig. 6) inside the cavity by pouring the first product 12 on top thereof.

The cosmetic article 20 was then coupled with a device 15 and extracted from the mould 10 as shown in Figure 7.

## Claims

1. Cosmetic article (20), preferably make-up article, in the form of a stick comprising:
- a first cosmetic product (12A), in the form of a stick of substantially solid or pasty consistency at room temperature, which defines at least one internal cavity (13); and
- a second cosmetic product (14), liquid at room temperature, housed in at least one internal cavity (13),
the cavity (13) providing a cross section with a shape configured to close itself mechanically during the use of the cosmetic article (20), minimizing or preventing a leak of the second cosmetic product (14) from the cavity itself after the use of the cosmetic article.

2. Cosmetic article (20) according to the preceding claim, wherein, at least at a first use, the cavity is totally surrounded by the first cosmetic product (12A), or by one or more products having a substantially solid or pasty consistency at room temperature.

3. The cosmetic article (20) according to claim 1, wherein a maximum thickness (S) of the cross section of the cavity (13) is between 1,0 and 3,5 mm, preferably between 1,8 and 2,8 mm, still more preferably of 2,2 mm.

4. Cosmetic article according to claim 1, wherein the second cosmetic product (14) contains solid decorative elements, or fat-soluble dye, or a combination thereof.

5. Cosmetic article according to claim 1, wherein said cavity (13) extends for at least half the height (A1) of said stick, the cross-sectional area of said internal cavity (13) being less than the area, in cross section, of said first product.

6. Cosmetic article according to claim 1, wherein the first cosmetic product (12A) is transparent or translucent at room temperature, so as to make the contents of said internal cavity (13) visible from the outside of the cosmetic article, to a user.

7. Cosmetic article according to claim 1, wherein said first cosmetic product (12A) comprises:
- a matrix comprising one or more of: tridecyl trimellitate, hydrogenated polyisobutene and/or octyldodecanol, esters, waxes, film formers;
- a gelling agent, comprising a mixture of dibutyl ethylhexanoyl glutamide and dibutyl lauroyl glutamide, dispersed in the lipid matrix.

8. The cosmetic article according to claim 1, wherein the second cosmetic product (14) comprises:
i) up to 95% by weight of water, based on the weight of the second product (14, 14A),
ii) solid decorative elements, or fat-soluble dye, or a combination thereof.

9. Process for the preparation of a cosmetic article (20), preferably of a make-up article, comprising the steps of:
i. providing a mold (10) comprising a cavity (22),
ii. insert a core (11) in the cavity (22) of the mold (10), so as to define a first casting cavity (16) between the walls of the mold (10) which define the cavity (22) and the external perimeter of the 'soul (11),
iii. pour a first cosmetic product (12) into the first casting cavity (16) and wait for said first product to solidify,
iv. once the first cosmetic product (12) has solidified, extract the core (11), so that the portion of the first solidified product (12A) which housed the core (11) defines an internal cavity (13) which provides a cross-section with a shape configured to close itself mechanically during use of the cosmetic article (20), minimizing or preventing a leak of the second cosmetic product (14) from the cavity itself after use of the cosmetic product,
v. pour a second cosmetic product (14), in substantially liquid form even at room temperature into the internal cavity (13),
vi. isolate the second product (14) liquid inside the internal cavity (13); And
vii. unmold the cosmetic article (20) from the mould.

10. The process of claim 9, wherein the second liquid product (14) inside the cavity (13) is isolated by pouring over it a product which assumes a substantially solid or pasty consistency at room temperature, preferably the first product (12) heated up to a liquid consistency.

11. The process of claim 9, wherein the internal cavity (13) has a cross section according to one of the following geometric figures: circle, ellipse, triangle, square, rectangle, heart-shaped, star-shaped.

12. The process of claim 9, wherein the core (11) is covered with a release agent before being inserted into the mold (10).
